# EUROPÄISCHE PATENTSCHRIFT

(11) **EP 2 714 186 B1**
(45) Veröffentlichungstag und Bekanntmachung des Hinweises auf die Patenterteilung: **19.08.2015**
(21) Anmeldenummer: 12740472.1
(22) Anmeldetag: 30.05.2012
(51) Int. Cl.: A61N 1/32, A61H 23/02, A61C 8/02, A61C 19/06

(54) **ANORDNUNG ZUR TOPISCHEN STIMULATION DER OSSIFIKATION/OSTEO-/SOFT-TISSUE-GENESE UND/ODER SUPPRESSION MIKROBIELLER INFLAMMATION SOWIE ZUR OSSEOINTEGRATION VON IMPLANTATEN**
ARRANGEMENT FOR TOPICAL STIMULATION OF OSSIFICATION/OSTEOGENESIS/SOFT TISSUE FORMATION AND/OR SUPPRESSION OF MICROBIAL INFLAMMATION, AND FOR OSSEOINTEGRATION OF IMPLANTS
DISPOSITIF POUR LA STIMULATION TOPIQUE DE L'OSSIFICATION/OSTÉOGENÈSE/GENÈSE DES TISSUS MOUS ET/OU L'ÉLIMINATION DE L'INFLAMMATION MICROBIENNE AINSI QUE POUR L'OSTÉO-INTÉGRATION D'IMPLANTS

(30) Priorität: 01.06.2011 DE 102011050813
(43) Veröffentlichungstag der Anmeldung: 09.04.2014
(73) Patentinhaber: Fraunhofer Gesellschaft zur Förderung der angewandten Forschung E.V., 80686 München (DE)
(72) Erfinder: LAUSCH, Holger, 07743 Jena (DE); ARNOLD, Michael, 07743 Jena (DE); BRAND, Michael, 07743 Jena (DE)
(74) Vertreter: Oehmke, Volker
(86) Internationale Anmeldenummer: PCT/DE2012/100161
(87) Internationale Veröffentlichungsnummer: WO 2012/163347

(56) Entgegenhaltungen:
- WO-A2-02/060522
- US-A1- 2009 048 647
- US-A1- 2010 143 871
- US-A1- 2011 125 212

## Beschreibung

Die Erfindung betrifft eine Anordnung zur topischen, bioelektrischen und/oder biomechanischen Stimulation der Ossifikation / Osteogenese von Knochen und Gelenken in der Unfallchirurgie / Orthopädie sowie die Osseointegration in der implantologischen Chirurgie.

Neben den in vivo im Einsatz befindlichen elektrostimulativen Implantaten gibt es unterschiedliche biomechanische Verfahren zur Knochenheilung und beanspruchungsgerechten Osseointegration. Die Stimulationsverfahren wirken dabei in der Regel ex vivo transkutan und werden zum Teil benutzt, um in vivo Stimulationsaktoren anzuregen. Letztere setzen einen, wie auch immer gearteten, definierten Energietransfer von ex vivo zu in vivo oder einen direkten chirurgischen Zugriff auf das Stimulationsgebiet voraus. Um die therapeutische Belastung des Patienten sowie die Kosten zu senken, werden immer mehr minimal- bis noninvasive Behandlungsmethoden bevorzugt. Im Bereich der Kieferchirurgie spielt dabei auch der ästhetische Aspekt eine Rolle. Die ex vivo basierten Stimulationsverfahren sind zudem mit hohen Anforderungen bezüglich der Einhaltung von Grenzwerten konfrontiert, um Nebenwirkungen, wie beispielsweise Gewebeerwärmung sowie allgemein die Schädigung von gesunden Knochen und Geweben zu vermeiden. Oberflächen- und strukturbasierte Verfahren, Anordnungen und Systeme zielen insbesondere auf die primäre und sekundäre Verankerung von Implantaten. Stabilität wird dabei im Bereich der Zahnimplantate in der Regel über atraumatisch und selbstschneidende Gewindeformen mit Schneidenuten erreicht. Die optimale Osteo(nen)anlagerung soll durch eine intelligente und/oder bioanaloge Oberflächengestaltung (Noppen-/Wellen-Strukturen, definierte Rauheit, Mikro-/Nano-Strukturen etc.) erreicht werden. Für die beschleunigte Osteo(nen)anlagerung werden auch spezielle pharmazeutische oder bioanaloge Wirkstoffbeschichtungen (Melatonin, Kollagen, etc.) der Oberflächen wie auch die Integration von Wirkstoffdepots in den Implantaten vorgeschlagen.

Neben einer Vielzahl von nacheinander und/oder temporär parallel wirkenden, prozessual notwendigen Wachstumsfaktoren (Anzahl größer 10 bis ca. 30) entscheiden auch die damit zusammenhängenden chemo-, mechano-, elektro-/galvano- und thermotaktischen bzw. -transduktiven Signal- und Transaktionsprozesse in Defekt- oder Regressionsregionen darüber, ob, wo und wie die Osteogenese stattfindet.

In zahlreichen Veröffentlichungen werden die unterschiedlichsten Stimulationsverfahren für die Osteogenese/Bone- und das Soft-Tissue-Management vorgestellt.

In der Regel sind sie aber mit minimalinvasiven oder invasiven Eingriffen verbunden. Beispiele für invasive Anordnungen von Sensoren, Aktoren und weiteren Systemkomponenten sind in der US 2010/0143871 A1 und der US 2011/0125212 A1 offenbart. So ist in dem Dokument US 2010/0143871 A1 ein Gerät zur elektrischen Stimulation eines Knochens beschrieben. Durch die elektrischen Stimulation sollen das Wachstum und die Heilung des Knochens gefördert werden. Das Gerät besteht unter anderem aus einem Zahnschraubenimplantat, das dazu dient, um mittels einer Schraube in einen Knochen eingeschraubt zu werden. Die US 2010/0125212 A1 betrifft ein Verfahren zur Modulation der Funktion der oberen Atemwege, indem Larynxmuskeln erste und zweite Therapiesignale appliziert werden. Die dazu erforderlichen Elektroden werden invasiv platziert.

In einigen Fällen werden Systemkomponenten implantiert, die wieder explantiert werden müssen. Die rein noninvasiven Verfahren, wie in der WO 02/060522 A2 vorgestellt, haben den Nachteil, dass die topisch einwirkende Energie - mechanisch, akustisch, optisch, elektrisch, elektromagnetisch, thermoelektrisch oder magnetisch - perkutan, d. h. transdermal oder transkutan in den Organismus eingebracht wird. Die bekannten Nachteile wie Gewebeerwärmung und/oder begrenzte Eindringtiefen bei im Transitbereich unschädlichen, vertretbaren Leistungen, führen meist zu erheblichen Einschränkung oder gar zum Ausschluss der zu erreichenden lokalen Reaktion.

Mit der US 2009/0048647 A1 wird ein Verfahren offenbart, bei dem Signale im Mundraum eines Patienten erfasst werde, die im weiteren lediglich als Auswahlkriterien für bereits vorgefertigte Antwortsignale aus einer Menge von Antwortsignalen dienen.

Die Effektivität hängt diesbezüglich einerseits vom Abstand der stimulativen oder suppressiven Systemkomponenten zum Ort der gewünschten lokalen Reaktion sowie andererseits vom gewählten Energie- und/oder Gradiententransferverfahren ab. In DE 11 2007 001 708 A5 und WO 2009/109172 A2 wird ein Verfahren zum topischen Energietransfer von ex vivo in Richtung in vivo, d. h. in situ, beschrieben, welches die oben genannten Nachteile nicht besitzt und somit auch draht- und berührungslos ohne Beeinflussung von Transitgeweben zum in situ Wirkbereich einen topisch genauen Energieeintrag und damit auch therapeutisch wirkende Gradienten erzeugen kann.

Bekannt aus der Forschung sind der Einfluss von an den Grenzflächen zwischen Knochen- und Weichteilgeweben, die extrazelluläre bzw. extrabakterielle Matrix sowie intern auf die Vaskulisierung und Angiogenese wirkenden physikalischen stimulativen oder suppressiven Gradienten, wie z. B.
- akustische oder mechanische Stosswellen oder Ultraschall (Mechanotransduktion) inkl. Vibration,
- positive wie negative Gravitation,
- Spaltsituationen (Kohäsion, Adhäsion),
- Oberflächenspannung/-energie an den Grenzflächen,
- elektrische Felder DC/AC - isoliert (V/cm),
- elektrische Felder DC/AC - leitend (A),
- elektromagnetische und/oder magnetische Gleich- und Wechselfelder, deren unmittelbare Wirkung auf Gewebe und ihre Wachstumsprozesse allerdings strittig ist und
- Chemotransduktion und andere Signaltaxien.

Der Erfindung liegt die Aufgabe zugrunde, eine Möglichkeit anzugeben, mit der ein adaptives und additives therapeutisches Verfahren auf der Basis einer einfach gestalteten und wirkungsvollen Sensor- und/oder Aktoranordnung zur noninvasiven Detektion realer elektrischer, mechanischer/akustischer und/oder thermischer Zustände und Gradienten in gesunden Gewebebereichen über Sensoren inklusive Signalverarbeitung sowie einer künstlichen signaltechnischen Erzeugung und topischen Applizierung therapeutisch unmittelbar und/oder mittelbar wirkender elektrischer, mechanischer/akustischer und/oder thermischer Gradienten in defekten oder geschädigten Gewebebereichen durchgeführt werden kann.

Erfindungsgemäß wird die Aufgabe mit einer Anordnung nach Anspruch 1 gelöst.

Eine erfindungsgemäß ausgestaltete Anordnung besteht darin, dass in einem individuell angepassten Kiefer-Zahnschutzsystem, deren Gaumenandruck/ -kontaktbereiche maximal vergrößert sind, die Sensoren und/oder Aktoren im Bereich des Unter- und/oder Oberkieferknochens derart integriert sind, dass dort biomechanische und/oder bioelektrische und/oder biothermische Signale im jeweiligen Kieferknochen-/Zahnwurzel-/Zahnbett-/Zahnfleisch-/Zahnhaltebereich sensitiv erfasst und topisch suppressiv und/oder stimulativ eingebracht werden können.

Vorteilhafterweise können akustisch/mechanisch piezoelektrisch, bioelektrisch, biothermisch und/oder biomagnetomechanisch wirkende Sensoren und/oder Aktoren zum Einsatz kommen.

Die Sensoren oder Aktoren können dabei drahtgebunden und/oder drahtlos und/oder berührungslos mit elektrischer Energie versorgt werden.

Mit der erfindungsgemäßen Anordnung wird eine beschleunigte Regeneration von Knochen-, Weich- und/oder Bindegewebe sowie die Generierung eines direkten funktionellen und strukturellen Verbund zwischen dem organisierten, lebenden Knochen- und/oder Weichteil- und/oder Neuronalgewebe und der Oberfläche eines belasteten Implantates erreicht.

Mit einer erfindungsgemäßen Anordnung sind dabei Gradientenwirkungen erzielbar, wie z. B.
- die Zellausrichtung orthogonal zu anliegenden elektrisch isolierten Gleichfeldern (Knochen- und Weichteilgewebe),
- das Zellwachstum inkl. Proliferation entlang mechanischen Hauptbelastungslinien,
- die reziproken Piezoeffekte in Knochengeweben über anliegende elektrisch isolierte Wechselfelder,
- die stimulativen Wirkungen von Vibrationen und/oder anliegenden elektrisch isolierten Wechselfeldern in Geweben,
- die suppressiven Wirkungen von Vibrationen und/oder anliegenden elektrisch isolierten Feldern auf die mikrobielle extrazelluläre Matrix - antibiotische Wirkungen,
- die gezielte Beeinflussung der Oberflächenspannung/-energie an den Gewebegrenzflächen durch künstlich herbeigeführte Spaltsituationen/-belastungen und
- die künstliche Simulation von Gewebebelastungssituationen etc. über elektrische Felder und mechanische Stimuli,
für die antibakterielle Prävention gegen den pathologischen und/oder traumatischen Gewebeabbau (Bone- und Soft-Tissue) sowie für das additive, generative Bone- und Soft-Tissue-Management allgemein.

Der Begriff Aktor soll nicht auf einen mechanisch bzw. schallbasiert wirkende Aktuator beschränkt sein, sondern vielmehr als Applikator verstanden werden, über den mechanische/akustische, elektrische, magnetische, thermische und/oder chemische Gradienten in die Gewebe appliziert werden können. Diese Applikation ist der Kern des additiven therapeutischen Verfahrens.

Die Erfindung soll nachfolgend anhand von Ausführungsbeispielen näher erläutert werden. In den dazugehörigen Zeichnungen zeigen:
- Fig. 1: eine schematische Darstellung des Verfahrensablaufs mittels erfindungsgemäßer Anordnung,
- Fig. 2: eine schematische Darstellung der Anordnung und
- Fig. 3: ein Kiefer- Zahnschutzsystem.

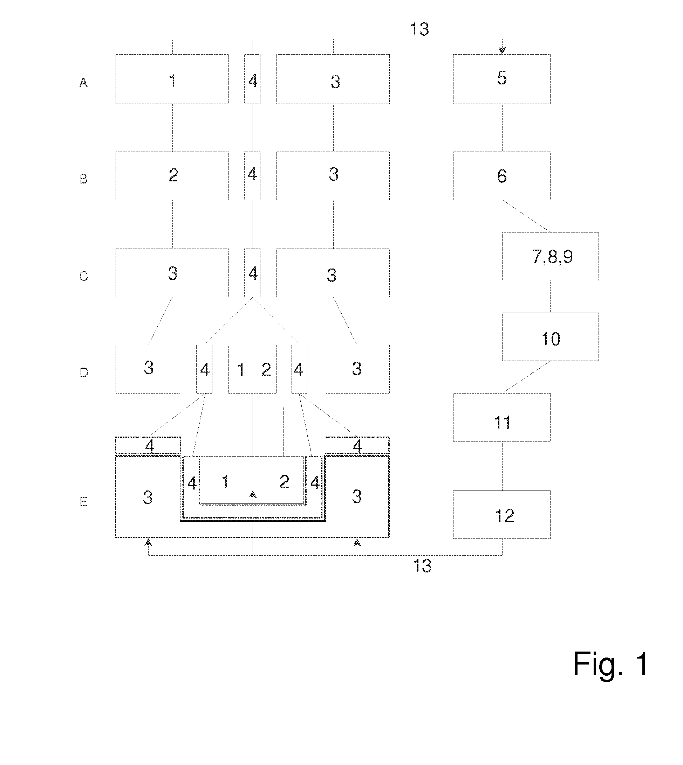

In Fig. 1 sind schematisch ein Implantat 1, ein Zahn 2, Knochen/Gewebe 3 und Grenzschichten 4 eines Probanden dargestellt. Ferner sind der erfindungsgemäßen Anordnung die Sensormodule 5, das Signalverarbeitungsmodul 6, das Signalgeneratormodul 11, die Aktormodule 12 sowie die Netzwerk/Cluster/Verbinder 13, welche ebenfalls nur schematisch dargestellt sind, zuzurechnen.

Die Sensormodule 5 nehmen Signale auf, die für die zu applizierenden stimulativ oder suppressiv wirksamen Gradienten (Stimuli) relevant und/oder für den Applikationsbereich signifikant sind. Dies geschieht vorzugsweise an ungeschädigten, mit dem Applikationsbereich vergleichbaren Bereichen, beispielsweise symmetrisch am Kiefer, ggf. zu Vergleichszwecken auch im zu behandelnden (geschädigten) Applikationsbereich. Dabei müssen Signalaufnahme 7 durch die Sensormodule 5 und Applikation durch die Aktormodule 12 keineswegs gleichzeitig und am selben Probanden erfolgen. Vielmehr ist auch eine Signalaufnahme 7 im Vorfeld eines geplanten Eingriffs am noch ungeschädigten Bereich oder an Vergleichsprobanden im Rahmen von Studien möglich, um die gewonnen Daten im Nachgang für den Applikationsvorgang zu benutzen. Die Sensormodule 5 geben die aufgenommenen Signale an das Signalverarbeitungsmodul 6 weiter. Hier erfolgt nach Signalaufnahme 7 die Signalverarbeitung 8 sowie die Vorkonditionierung der zu applizierenden Gradienten durch Bestimmung/Vergleich 9 mit anschließender Signaloptimierung 10 und ggf. der Speicherung/Aktualisierung der optimierten/aufgenommenen Applikationsparameter zur zeitlich nachgelagerten Verwendung. Diese optimierten Signale bzw. zur Signalgenerierung erforderlichen Applikationsparameter werden den Signalgeneratormodulen 11 zugeführt, die wiederum anschließend die Aktormodule 12 ansteuern. In Abhängigkeit vom Applikationsziel können unterschiedliche Typen von Aktormodulen 12 eingesetzt werden, die in der Lage sind, unterschiedliche Kombinationen akustischer, mechanischer, elektrischer, elektromagnetischer, optischer, thermischer oder chemischer Stimuli in den Applikationsbereich einzubringen. Dabei ist es keineswegs erforderlich, dass sich Signalgeneratormodule 11 und Aktormodule 12 am selben Ort befinden und/oder direkt miteinander gekoppelt sind, vielmehr können die zu applizierenden Stimuli auch fernwirksam, z. B. durch Felder, von den Signalgeneratormodulen 11 auf die Aktormodule 12 übertragen werden. Die hier zum Einsatz kommenden Sensoren 5 sind Piezomodule mit integrierten Tempertatursensoren. Die Temperatur ist ein wichtiger Faktor bei der Identifizierung biologischer Prozessabläufe. Da es keine Thermopiles gibt, welche wechselseitig als Peltier- oder Seebeck-Element geschaltet werden können, ist eine Einflussnahme auf die Temperatur nicht möglich. Die Piezomodule können wechselseitig als Sensor 5 zur Aufnahme von Vibrationen, Puls und Blutfluss oder als Aktor 12 zur Simulation der genannten mechanischen Komponenten fungieren.

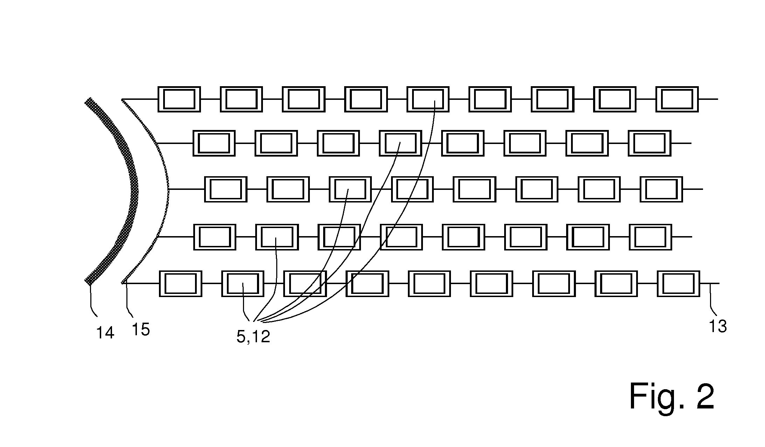

Die in Figur 2 dargestellten, dem therapeutischen Zielgebiet quasi übergestülpten, dreidimensionalen Sensor-/Aktor-Cluster ermöglichen somit die Generierung einer virtuellen dreidimensionalen Zellmatrix (Bone-/Soft-Tissue), auf deren Basis am Zielgebiet geeignet ausgerichtete Gradienten direkt bzw. resultierend als Summe bzw. Wechselwirkung der jeweils beteiligten Cluster-Aktoren den Auf- bzw. Wiederaufbau zerstörten bzw. beschädigten Gewebes 3 zielgerichtet appliziert werden. Mittels eines mehrmaligen Ablaufens der beiden Hauptverfahrensschritte Detektion-Signalverarbeitung und Signalgenerierung-Applizierung ist ein Monitoring der aktuellen Gewebebildung bzw. -abbaus und damit eine Anpassung der topischen Einbringung der Gradienten in das lokale Zielgewebe in geeigneter, anpassbarer topisch Stärke und dreidimensionaler Ausrichtung möglich.

Ein mehrmaliges Ablaufen der beiden Hauptverfahrensschritte Detektion-Signalverarbeitung und Signalgenerierung-Applizierung gilt ebenso auch bezogen auf bereits o. g. Vorteile, wie
- die Zellausrichtung orthogonal zu anliegenden elektrisch isolierten Gleichfeldern (Knochen- und Weichteilgewebe),
- das Zellwachstum inkl. Proliferation entlang mechanischen Hauptbelastungslinien,
- die reziproken Piezoeffekte in Knochengeweben über anliegende elektrisch isolierte Wechselfelder,
- die stimulativen Wirkungen von Vibrationen und/oder anliegenden elektrisch isolierten Wechselfeldern in Geweben,
- die suppressiven Wirkungen von Vibrationen und/oder anliegenden elektrisch isolierten Feldern auf die mikrobielle extrazelluläre Matrix - antibiotische Wirkungen,
- die gezielte Beeinflussung der Oberflächenspannung/-energie an den Gewebegrenzflächen durch künstlich herbeigeführte Spaltsituationen/belastungen und
- die künstliche Simulation von Gewebebelastungssituationen etc. über elektrische Felder und mechanische Stimuli sowie
- für die antibakterielle Prävention, gegen den pathologischen und/oder traumatischen Gewebeabbau (Bone- und Soft-Tissue) sowie für das additive, generative Bone- und Soft-Tissue-Management allgemein.

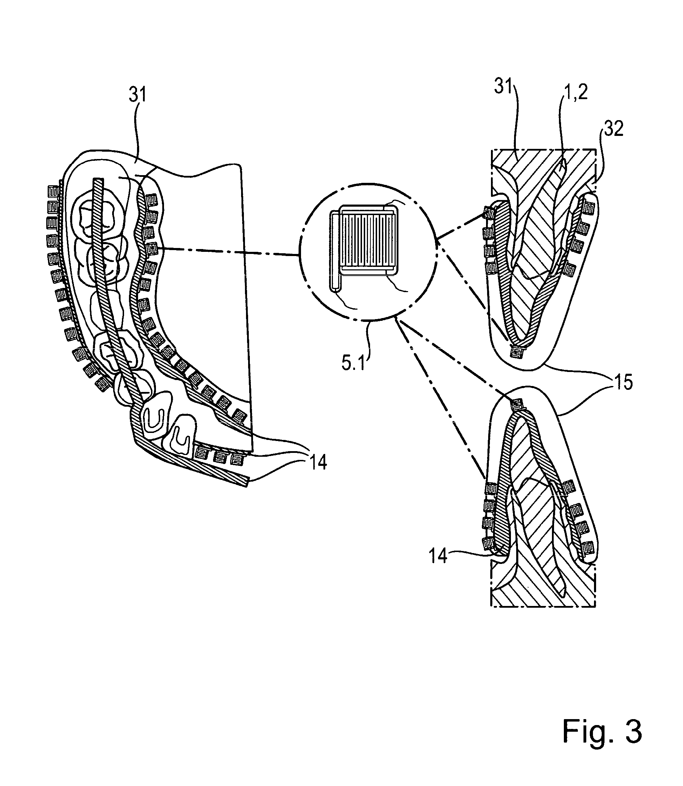

Bezogen auf den exemplarischen dentalen Anwendungsfall soll auf der Basis eines jeweiligen zahnärztlichen Kieferabdruckes ein individuell oder normiert über CAD/CAM, Sägeschnittmodellen generiertes, modifiziertes und integriertes elastisches Kiefer-Zahnschutzsystem 15 (Fig. 3) für die Maxilla und Mandibula in Anlehnung an bekannte Kieferschutzsysteme aus dem Sport verwendet werden, deren Oberfläche nicht nur transversal der Maxilla und Mandibula folgend im unmittelbaren Zahnbereich, sondern auch kranial und kaudal sowie lateral und medial dem jeweiligen Kieferknochen-/Alveolarbereich folgend anliegt. Traumatisch oder extraktionsbedingte Leerstellen können dabei ausgefüllt werden. Der Zwischenraum zwischen dem elastisches Kiefer-Zahnschutzsystem 15 und den Kieferweichteilgeweben kann ggfs. mit einer geeigneten λ/4 - Gelschicht 14, Fig. 2 und Fig. 3 ausgefüllt werden.

Über bzw. unter den Zähnen 2 bzw. ehemaligen Zahnstellen (nach Extraktion oder traumatischem Verlust entstanden), sowie an den gegenüberliegenden unteren und oberen sowie inneren und äußeren Gaumenbeinansatz-/Alveolarbereichen sind in regelmäßigen Abständen der Zahnlinie (Maxilla und Mandibula) folgend untereinander drahtgebunden Taschen für Sensoren 5 und/oder Aktoren 12 angeordnet, in denen thermische, piezoelektrische und/oder kapazitive Sensoren 5, Aktoren 12 und ggf. Elektroden Fig. 2 bzw. Fig. 3 platziert werden können. Diese Sensoren 5 und Aktoren 12 können nebeneinander und übereinander in einer oder mehreren Reihen als Netzwerk/Cluster/Verbinder 13, gemäß Fig. 3 angeordnet und in geeigneter Weise schaltungstechnisch mit einer externen oder internen Signalverarbeitungseinheit 6, Figur 1, verbunden werden, in der die detektierten körpereigenen Signale (Frequenz, Amplitude) aus gesunden und geschädigten Bereichen aufbereitet, mit Signalen aus gespiegelt gesunden Bereichen und/oder mit einer vorher erstellten Signalbibliothek 7, 8, 9 - Fig. 1 verglichen und so verarbeitet werden, dass sie in einer nachgelagerten internen und/oder externen Signalgeneratoreinheit 11 - Fig. 1 als therapeutische wirkende Signale generiert, an die Aktoren 12 - Fig. 1, 2, 3 im elastischen Kiefer-Zahnschutzsystem 15 - Fig. 1, 2 weitergeleitet und topisch vor Ort appliziert werden.

Die Sensor- und Aktorenanordnungen 5, 12 inkl. Signalverarbeitung 8 und Signalgenerierung 11 können dabei räumlich und zeitlich zusammen- oder auseinanderfallen Gleiches gilt auch bezogen auf das Verfahren, d.h. die beiden Hauptverfahrensschritte Detektion-Signalverarbeitung und Signalgenerierung-Applizierung.

Als Sensoren 5 und Aktoren 12 bzw. Transducer können ggf. gleiche elektrische/elektronische Bauteile wie gehauste Miniatur-Piezoaktoren in Multilayer-Bauweise gekoppelt mit separat gehausten kapazitiven, flächigen oder mehrlagigen Elektroden (z.B. gedruckte Gold- oder Kupferpasten) verwendet werden.

Die Netzwerk/Cluster/Verbinder 13 der Sensoren/Aktoren 5, 12 Fig. 2 und Fig. 3 können räumlich ein- oder mehrreihig regelmäßig und/oder versetzt angeordnet werden, um beliebige dreidimensionale Feldformen und/oder physikalische Gradienten zu detektieren und/oder zu applizieren.

Durch die Wechselwirkung der beiden Hauptverfahrensschritte Detektion-Signalverarbeitung und Signalgenerierung-Applizierung kann ein Monitoring der aktuellen Gewebebildung bzw. des Gewebeabbaus und damit eine Anpassung der topischen Einbringung der therapeutischen Gradienten in das lokale Zielgewebe in geeigneter, anpassbarer Stärke und dreidimensionaler Ausrichtung erfolgen.

Anwendungsfelder dieser Erfindung sind in Gestalt angepasster Sensor-/Aktoranordnungen 5, 12 mit internen/in vivo/in situ und/oder externen/ex vivo Signalverarbeitungs- und Signalgeneratoreinheiten 6, 11 inkl. drahtlosen und/oder drahtgebundenen Energie- und Signalübertragungssystemen generell alle Knochen- und Weichteilgewebeverbünde, Gelenk-, Prothesen- und Implantatbereiche im menschlichen und tierischen Organismus.

Mit der erfindungsgemäßen Anordnung kann insbesondere ein Verfahren zur topischen, bioelektrischen und oder biomechanischen Stimulation der Ossifikation/ Osteogenese von Knochen/Geweben 3 und Gelenken durchgeführt werden, indem die in einem individuell anpassbarem, erweiterten elastischen Kiefer-Zahnschutzsystem 15 integrierten Sensoren 5 im Bereich des Unter- und oder Oberkieferknochens die biomechanischen und oder bioelektrischen und oder biothermischen Signale sowie ihre Dynamik im jeweiligen Kieferknochen-/Zahnwurzel-/Zahnbett-/Zahnfleisch-Zahnhaltebereich sensitiv erfassen und an die Signalverarbeitungseinheit 6 weiterleiten, diese Signale analysiert und in der Signalgeneratoreinheit 11 adaptiert und therapeutisch optimiert und die generierten Stimulationssignale im jeweiligen Kieferknochen-/Zahnwurzel-/Zahnbett-/Zahnfleisch-Zahnhaltebereich, vorzugsweise Zahnimplantat- und oder Zahnlockerungsbereich topisch suppressiv und oder stimulativ appliziert werden.

Das Verfahren ist insbesondere dadurch gekennzeichnet, dass die funktional topisch platzierbaren Aktoren 12 im individuell anpassbaren, erweiterten elastischen Kiefer-Zahnschutzsystem 15 in ebenfalls topisch definierbaren Bereichen des Kieferknochen bzw. vorzugsweise im Bereich von primär verankerten Zahnimplantaten und oder gelockerten Zahnhalteapparaten von der Signalgeneratoreinheit 11 erzeugte, definierte biomechanische, biothermische und oder bioelektrische Stimulations- und oder Suppressionssignale applizieren.

Das Verfahren zeichnet sich weiterhin dadurch aus, dass die topisch applizierten Signale in den Spaltbereichen zwischen Zahn-/Zahnimplantat und oder Knochenersatzstoffen und oder Kieferknochen bzw. Zahnhalteapparat funktional die Ossifikation/ Osteogenese inkl. Osseointegration anregen und unterstützen.

Die Frequenz und Amplitude der applizierten bioelektrischen und biomechanischen, therapeutischen Signale können entsprechend der jeweiligen Spaltsituation (Größe, Lage, Ausdehnung, Form) konstant, variierend und oder durchmodulierender Natur sein.

Die applizierten Signalen können den Kieferknochen und oder jeweiligen Zahnhalteapparat (Zahn/Implantat) dergestalt bioaktiv stimulieren, dass an und um den jeweiligen gegenüberliegenden Oberflächen der Spaltbereiche zwischen Kieferknochen, Zahnhalteapparat und/oder Zahn/Zahnimplantat die Nährstoff-, Blut- und Sauerstoffversorgung unterstützend bzw. initialisierend unterstützend stimuliert wird, um diesen Bereich mit Thrombozyten, mesenchymalen Vorläuferzellen und Fibroblasten sowie körpereigenen Wachstumsfaktoren und Proteinen für Osteogenesen, z.B. Osteoblastenausdifferenzierung etc., zu versorgen.

Mit dem Verfahren kann erreicht werden, dass die Fibromatrix zur Anlagerung, Proliferation und Ausdifferenzierung der mesenchymalen Vorläuferzellen zu Osteoblasten, die Calzium- und Collagenproduktion und somit auch die Schichtbildung stimuliert wird, indem biochemisch funktionalisierend körpereigene Stoffwechsel- und Wachstumsprozesse unterstützt werden.

Es ist insbesondere möglich, dass die mittels Vibrationen und oder anliegenden elektrisch isolierten Felder suppressive Gradienten auf die bakteriellen Biofilm mit antibiotischer Wirkung erzeugt werden.

Über die Einbringung bzw. Implantation von Implatatbasements, resorbierbaren Knochenzement, Schäumen und anderen dreidimensional zellulär besiedelbaren Strukturkörpern und oder -beschichtungen sowie durch die gezielte Beeinflussung der Oberflächenspannung/-energie an den Gewebegrenzflächen und oder durch künstlich herbeigeführte Spaltsituationen/-belastungen kann die Ossifikation/ Osteogenese von Knochen, Geweben und Gelenken topisch stimuliert und über Signaltransduktion ausgerichtet werden.

Selbst bei Ruhigstellung und oder Fixierung von Gewebebereichen wird eine künstliche Simulation von ursprünglich belastungs- und bewegungsabhängigen Gewebesignale über elektrische Felder und mechanische Stimuli und andere Gradienten ermöglicht und ein pathologisch und oder traumatisch und oder durch Nichtbelastung bedingter Gewebeabbau verhindert.

Durch die beiden Hauptverfahrensschritte Detektion-Signalverarbeitung und Signalgenerierung-Applizierung kann ein Monitoring der aktuellen Gewebebildung bzw. Gewebeabbaus und damit eine Anpassung der topischen Einbringung der therapeutischen Gradienten in das lokale Zielgewebe in geeigneter, anpassbarer topisch Stärke und dreidimensionaler Ausrichtung erfolgen.

Die Erfindung erschöpft sich nicht in dem vorstehenden Ausführungsbeispiel. Sie ist weder an eine bestimmte Anzahl, Art und Anordnung noch an eine bestimmte Form gebunden. Die genannten Merkmale können fachgerecht in andere Anwendungsbeispiele eingeführt werden. Auch sind unterschiedliche Kombinationen von vorstehend genannten Merkmalen zu neuen Ausführungsformen möglich. Überhaupt können alle in dieser Beschreibung und in den Ansprüchen genannten sowie in den Zeichnungen dargestellten Merkmale sowohl einzeln als auch in beliebiger Kombination miteinander erfindungswesentlich sein. Es muss nur gewährleistet sein, dass der/die Sensoren 5 und Aktoren 12 sich in einer Lagerungsstruktur befinden, die in einer definierten Umgebung mechanische, akustische, optische, elektrische, elektromagnetische, thermische, chemische oder magnetische Signale und/oder Impulse detektieren und entsprechende generier- und applizierbare Signale und/oder Impulsgebungen zulassen.

### Bezugszeichenliste

- 1: Implantat
- 2: Zahn
- 3: Knochen/Gewebe(soft)
- 4: Grenzschichten
- 5: Senoren/Sensormodule/Sensoranordnung
- 6: Signalverarbeitungsmodul/Signalverarbeitungseinrichtung
- 7: Signalaufnahme
- 8: Signalverarbeitung
- 9: Bestimmung/Vergleich
- 10: Signaloptimierung
- 11: Signalgeneratormodule/Signalgeneratoreinrichtung
- 12: Aktoren/Aktormodule/Aktorenanordnung
- 13: Netzwerk/Cluster/Verbinder
- 14: Gelschicht
- 15: elastisches Kiefer-Zahnschutzsystem
- 31: Knochengewebe
- 32: Weichgewebe
- A-E: Fallbeispiele

## Patentansprüche

1. Anordnung zur topischen Stimulation der Ossifikation/Osteo-/Soft-Tissue-Genese und/oder Suppression mikrobieller Inflammation sowie zur Osseointegration von Implantaten mit:
- Sensoren (5) und Aktoren (12), die in definierten Bereichen um einen Knochen-, Gewebe- und/oder neuronalen Defekt traumatischen Ursprungs noninvasiv und entsprechend einer definierten Grundordnung fest zueinander angeordnet sind, wobei
- mittels der Sensoren (5) bioelektrische, biomechanische, biochemische und/oder biothermische körpereigene Signale erfassbar sind und
- die Aktoren (12) dazu geeignet sind, generierte Signale topisch zu applizieren,
- einem Signalaufbereitungssystem, das eine Signalverarbeitungseinrichtung (6) und eine Signalgeneratoreinrichtung (11) aufweist, dadurch charakterisiert, dass
durch die Signalverarbeitungseinrichtung (6) die durch den Sensor (5) erfassten Signale verarbeitet und der Signalgeneratoreinrichtung (11) zugeführt werden und durch die Signalgeneratoreinrichtung (11) aus den zugeführten Signalen zu applizierende Signale generiert und an die Aktoren (12) weitergeleitet werden, so dass bioelektrische, biomechanische, biochemische und/oder biothermische und/oder andere körperähnliche Energie in Gestalt von Feldern, Impulsen und Gradienten topisch suppressiv oder stimulativ appliziert wird.

2. Anordnung nach Anspruch 1, **dadurch gekennzeichnet, dass** in einem individuell anpassbarem elastischen Kiefer-Zahnschutzsystem (15) die Gaumenandruck/- kontaktbereiche maximal vergrößert und darin die Sensoren (5) und/oder Aktoren (12) im Bereich des Unter- und/oder Oberkieferknochens so integriert sind, dass dort biomechanische und/oder bioelektrische und/oder biothermische Signale im jeweiligen Kieferknochen-/Zahnwurzel-/Zahnbett-/Zahnfleisch-/Zahn-haltebereich sensitiv erfasst und topisch suppressiv und/oder stimulativ eingebracht werden können.

3. Anordnung nach Anspruch 1 oder 2, **dadurch gekennzeichnet, dass** die integrierten Sensoren (5) und Aktoren (12), akustisch/mechanisch piezoelektrisch, bioelektrisch, biothermisch und/oder biomagnetomechanisch wirkende Sensoren (5) und Aktoren (12) sind.

4. Anordnung nach einem der vorhergehenden Ansprüche, **dadurch gekennzeichnet, dass** die Sensoren (5) oder Aktoren (12) kabel-/drahtgebunden und/oder drahtlos mit einer ex vivo Sensor- und/oder Aktor-Signalverarbeitungs-(6) und/oder Signalgeneratoreinheit (11), die integraler Bestandteil des Signalaufbereitungssystems ist, verbunden sind.

5. Anordnung nach Anspruch 2 und 4, **dadurch gekennzeichnet, dass** die in dem elastischen Kiefer-Zahnschutzsystem (15) integrierten Sensoren (5) zur Weiterleitung von den registrierten biomechanischen, biothermischen und/oder bioelektrischen Signalen in definierten Kieferknochenbereichen mit der ex vivo Sensor- und/oder Aktor-Signalverarbeitungs- (6) und/oder Signalgeneratoreinheit (11) verbunden sind.

6. Anordnung nach Anspruch 2, **dadurch gekennzeichnet, dass** die Sensoren (5) und Aktoren (12) elastischen des Kiefer-Zahnschutzsystems (15) drahtgebunden und/oder drahtlos und/oder berührungslos mit elektrischer Energie versorgt werden.

7. Anordnung nach Anspruch 2, **dadurch gekennzeichnet, dass** topisch im Bereich der Sensoren (5) und/oder Aktoren (12) in dem elastischen Kiefer-Zahnschutzsystem (15) ex vivo berührungslos magnetische Wechselfelder applizierbar sind, die über ein in vivo induktives Generatorsystem mit einem mit mindestens einer Spule umwickelten statischen und/oder in mindestens einer Richtung frei rotierenden, magnetisierbaren Kernkörper in elektrische Energie umgewandelt werden.

8. Anordnung nach Anspruch 5, **dadurch gekennzeichnet, dass** die ex vivo Sensor- und/oder Aktor-Signalverarbeitungs- (6) und/oder Signalgeneratoreinheit (11) integraler Bestandteil des elastischen Kiefer-Zahnschutzsystems (15) ist.

## Claims

1. Arrangement for topical stimulation of ossification/osteogenesis/soft tissue formation and/or suppression of microbial inflammation, and for osseointegration of implants, comprising:
- sensors (5) and actuators (12), which are arranged in fixed relation to one another in defined regions around a bone defect, tissue defect and/or neuronal defect of traumatic origin in a non-invasive manner and in accordance with a defined basic arrangement, wherein
- bioelectrical, biomechanical, biochemical and/or biothermal endogenous signals can be detected by means of the sensors (5), and
- the actuators (12) are suitable for topically applying generated signals,
- a signal processing system which has a signal processing unit (6) and a signal generator unit (11), **characterised in that**
- by means of the signal processing unit (6) the signals detected by the sensor (5) are processed and are supplied to the signal generator unit (11) and by means of the signal generator unit (11) signals to be applied are generated from the signals supplied and are transmitted to the actuators (12), so that bioelectrical, biomechanical, biochemical and/or biothermal and/or other body-like energy in the form of fields, pulses and gradients is applied topically suppressively or stimulatively.

2. Arrangement according to claim 1, **characterised in that** in an individually adaptable elastic jaw/tooth protection system (15) the palate contact pressure/contact regions are enlarged to a maximum and the sensors (5) and/or actuators (12) are integrated therein in the region of the lower and/or upper jaw bone in such a way that biomechanical and/or bioelectrical and/or biothermal signals can be detected there in a sensitive manner in the respective jaw bone/tooth root/tooth socket/gum/tooth supporting region and can be applied topically suppressively or stimulatively.

3. Arrangement according to claim 1 or 2, **characterised in that** the integrated sensors (5) and actuators (12) are acustically/mechanically piezoelectrically, bioelectrically, biothermally and/or biomagnetomechanically acting sensors (5) and actuators (12).

4. Arrangement according to one of the preceding claims, **characterised in that** the sensors (5) or actuators (12) are connected by a cable/wire and/or wirelessly to an ex vivo sensor and/or actuator signal processing unit (6) and/or signal generator unit (11) which is an integral component of the signal processing system.

5. Arrangement according to claim 2 and 4, **characterised in that** the sensors (5) integrated in the elastic jaw/tooth protection system (15) are connected to the ex vivo sensor and/or actuator signal processing unit (6) and/or signal generator unit (11) in defined jaw bone regions for transmitting the registered biomechanical, biothermal and/or bioelectrical signals.

6. Arrangement according to claim 2, **characterised in that** the sensors (5) and actuators (12) of the elastic jaw/tooth protection system (15) are supplied with electric energy by a wire and/or wirelessly and/or contact-free.

7. Arrangement according to claim 2, **characterised in that** in the region of the sensors (5) and/or actuators (12) in the elastic jaw/tooth protection system (15) alternating magnetic fields can be applied topically and ex vivo contact-free, which are converted into electric energy via an in vivo inductive generator system having a static core body with at least one coil wrapped around it and/or a magnetisable core body freely rotating in at least one direction.

8. Arrangement according to claim 5, **characterised in that** the ex vivo sensor and/or actuator signal processing unit (6) and/or signal generator unit (11) is an integral component of the elastic jaw/tooth protection system (15).

## Revendications

1. Dispositif pour la stimulation topique de l'ossification/ostéogenèse/genèse des tissus mous et/ou l'élimination de l'inflammation microbienne ainsi que pour l'ostéointégration d'implants, comprenant:
- des capteurs (5) et actionneurs (12), qui sont disposés de manière fixe les uns par rapport aux autres dans des régions définies autour d'un défaut osseux, défaut de tissu et/ou défaut neuronal d'origine traumatique d'une manière non invasive et conformément à un arrangement de base défini, dans lesquels
- des signaux endogènes bioélectriques, biomécaniques, biochimiques et/ou biothermiques peuvent être détectés au moyen des capteurs (5), et
- les actionneurs (12) conviennent à appliquer topiquement des signaux générés,
- un système de traitement de signal ayant une unité de traitement de signal (6) et une unité de générateur de signal (11), **caractérisé en ce que**
- au moyen de l'unité de traitement de signal (6) les signaux détectés par le capteur (5) sont traités et délivrés à l'unité de générateur de signal (11) et au moyen de l'unité de générateur de signal (11) des signaux à appliquer sont générés des signaux délivrés et sont transmis aux actionneurs (12), de telle façon que de l'énergie bioélectrique, biomécanique, biochimique et/ou biothermique et/ou d'autre énergie semblable à celle présente dans le corps humain sous forme de champs, impulsions et gradients est appliquée topiquement pour l'élimination ou la simulation.

2. Dispositif selon la revendication 1, **caractérisé en ce que** dans un système élastique de protection de mâchoire/dent (15) qui est adaptable individuellement les zones de pression de contact/de contact de palais sont élargies à un maximum et les capteurs (5) et/ou actionneurs (12) sont intégrés là-dedans dans la zone de l'os mandibulaire et/ou maxillaire de telle façon que des signaux biomécaniques et/ou bioélectriques et/ou biothermiques peuvent être détectés d'une manière sensible là dans la zone particulière d'os de mâchoire/de racine de la dent/de parodonte/de gencives/de soutien de la dent et peuvent être appliqués topiquement pour l'élimination ou la simulation.

3. Dispositif selon la revendication 1 ou 2, **caractérisé en ce que** les capteurs (5) et actionneurs (12) intégrés sont des capteurs (5) et actionneurs (12) agissants acoustiquement/mécaniquement piézo-électriquement, bioélectriquement, biothermiquement et/ou biomagnétomécaniquement.

4. Dispositif selon une des revendications précédentes, **caractérisé en ce que** les capteurs (5) ou actionneurs (12) sont reliés par câble/fil et/ou sans fil à une unité de traitement de signal (6) de capteur et/ou d'actionneur ex vivo et/ou une unité de générateur de signal (11) ex vivo qui est une partie intégrante du système de traitement de signal.

5. Dispositif selon la revendication 2 et 4, **caractérisé en ce que** les capteurs (5) intégrés dans le système élastique de protection de mâchoire/dent (15) sont reliés à l'unité de traitement de signal (6) de capteur et/ou d'actionneur ex vivo et/ou l'unité de générateur de signal (11) ex vivo dans des zones définies d'os de mâchoire pour transmettre les signaux registrés biomécaniques, biothermiques et/ou bioélectriques.

6. Dispositif selon la revendication 2, **caractérisé en ce que** les capteurs (5) et actionneurs (12) du système élastique de protection de mâchoire/dent (15) sont alimentés d'énergie électrique par fil et/ou sans fil et/ou sans contact.

7. Dispositif selon la revendication 2, **caractérisé en ce que** dans la zone des capteurs (5) et/ou actionneurs (12) dans le système élastique de protection de mâchoire/dent (15) des champs magnétiques alternatifs peuvent être appliqués topiquement et de façon ex vivo sans contact, qui sont transformés en énergie électrique par un système de générateur inductif in vivo ayant un corps de coeurs statique enroulé par au moins une bobine et/ou un corps de coeurs magnétisable tournant librement dans au moins une direction.

8. Dispositif selon la revendication 5, **caractérisé en ce que** l'unité de traitement de signal (6) de capteur et/ou d'actionneur ex vivo et/ou l'unité de générateur de signal (11) est une partie intégrante du système élastique de protection de mâchoire/dent (15).
